(19) **Europäisches Patentamt / European Patent Office / Office européen des brevets**

(11) **EP 3 368 005 B1**

(12) # EUROPEAN PATENT SPECIFICATION

(45) Date of publication and mention
of the grant of the patent:
**11.05.2022 Bulletin 2022/19**

(21) Application number: **16860635.8**

(22) Date of filing: **25.10.2016**

(51) International Patent Classification (IPC):
*A61K 8/64* (2006.01)   *A61Q 11/00* (2006.01)
*A61K 8/34* (2006.01)   *A61K 38/01* (2006.01)
*A61K 8/21* (2006.01)   *A61K 8/41* (2006.01)
*A61P 1/02* (2006.01)

(52) Cooperative Patent Classification (CPC):
**A61K 8/645; A61K 38/011; A61P 1/02; A61Q 11/00**

(86) International application number:
**PCT/US2016/058717**

(87) International publication number:
**WO 2017/074960 (04.05.2017 Gazette 2017/18)**

(54) **ORAL CARE PRODUCTS AND METHODS**

MUNDPFLEGEPRODUKTE UND -VERFAHREN

PRODUITS DE SOIN BUCCAL, ET PROCÉDÉS ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.10.2015 US 201562246165 P
26.10.2015 US 201562246163 P
26.10.2015 US 201562246161 P
30.10.2015 US 201562248992 P**

(43) Date of publication of application:
**05.09.2018 Bulletin 2018/36**

(73) Proprietors:
• **Colgate-Palmolive Company
New York, NY 10022 (US)**
• **BASF SE
67054 Ludwigshafen (DE)**

(72) Inventors:
• **KILPATRICK-LIVERMAN, LaTonya
Princeton, New Jersey 08542 (US)**
• **QIU, Jianhong
Warren, New Jersey 07059 (US)**
• **ZAIDEL, Lynette
Cranford, New Jersey 07016 (US)**
• **SUBKOWSKI, Thomas
69198 Schriesheim (DE)**

• **JENEWEIN, Stefan
67434 Neustadt (DE)**
• **KAROS, Marvin
68723 Plankstadt (DE)**
• **BOLLSCHWEILER, Claus
69118 Heidelberg (DE)**
• **WENDEL, Volker
64342 Seeheim-Jugenheim (DE)**
• **SCHNEIDER, Nina
68535 Edingen-Neckarhausen (DE)**
• **SANTARPIA, Ralph Peter
Edison, New Jersey 08820 (US)**

(74) Representative: **Sonnenhauser, Thomas Martin
Wuesthoff & Wuesthoff
Patentanwälte PartG mbB
Schweigerstraße 2
81541 München (DE)**

(56) References cited:
WO-A1-2005/023210    US-A1- 2006 222 602
US-A1- 2006 222 602    US-A1- 2007 116 831
US-A1- 2007 128 130    US-A1- 2008 160 091
US-A1- 2009 042 169    US-A1- 2010 196 287
US-A1- 2010 330 002

**Description**

**ORAL CARE PRODUCTS AND METHODS**

This application claims priority to the following applications: U.S. Provisional Application Serial No. 62/248992 filed 30 October 2015; U.S. Provisional Application Serial No. 62/246161 filed 26 October 2015; U.S. Provisional Application Serial No. 62/246163 filed 26 October 2015; and U.S. Provisional Application Serial No. 62/246165 filed 26 October 2015.

**BACKGROUND**

Dental enamel is a thin, hard layer of calcified material that covers the crown of teeth. The major mineral component of dental enamel is hydroxyapatite, a crystalline form of calcium phosphate. Chemical erosion of dental enamel may arise from tooth exposure to acidic food and drinks or to stomach acids arising from gastric reflux. The erosion of dental enamel can lead to enhanced tooth sensitivity due to increased exposure of the dentin tubules and increased dentin visibility leading to the appearance of more yellow teeth. The salivary pellicle (a thin layer of salivary glycoproteins deposited on teeth) is integral in protecting the teeth against an erosive challenge. As a result, people that experience xerostomia are more susceptible to acid erosion damage.

Existing methods developed to help prevent enamel erosion include incorporating a source of free fluoride into oral care compositions. Fluoride reduces damage to the enamel, through the formation of fluorapatite, which dissolves at a lower pH than hydroxyapatite and so is more resistant to acid damage and by maintaining a supersaturated environment within the plaque fluid surrounding the tooth that minimizes tooth demineralization. Stannous salts have also been incorporated into dentifrice formulations to protect the enamel surface similarly, by forming a more acid resistant mineral layer. Polymers have also been described that coat and protect the enamel surface.

Acids are also generated in the oral cavity when plaque containing cariogenic bacteria metabolize carbohydrates. Since plaque forms a barrier controlling the kinetics of proton and mineral diffusion through the enamel, plaque acids cause carious lesions. Incorporating fluoride ions in dentifrice formulations is the most common method to mitigate the effects of plaque acids. Fluoride reduces the rate of demineralization and enhances remineralization. Several approaches have also been developed to stabilize calcium phosphate salts or control the plaque pH to enhance remineralization.

Although methods have been developed to mitigate the effects of non-bacteria and bacteria generated acids on the teeth, there is still the need to provide improved oral care compositions that effectively repair the enamel from the effects of acid erosion and bacteria acids. US 2010/0196287 A1 discloses compositions, in particular washing and cleaning agents as well as cosmetic and pharmaceutical preparations, comprising perhydrolases and alkylene glycol diacetates. US 2006/0222602 A1 discloses and oral and dental hygiene product comprising a) a composite material containing poorly water-soluble calcium salts in the form of nanoparticulate primary particles which are between 5 and 150 nm long and have a cross-section of between 2 and 50 nm, and protein constituents selected from proteins, protein hydrolysates and protein hydrolysate derivatives, and b) between 0.1 and 9 wt. % of a cleaning agent, in relation to the total weight of the product.

**BRIEF SUMMARY**

The present inventors have unexpectedly found that partially hydrolyzed plant proteins, e.g., partially hydrolyzed proteins from wheat, rice, almond, potato, soya, pea or combinations thereof, for example partially hydrolyzed cereal proteins (hydrolyzed proteins from grains of the family *Poaceae*, for example partially hydrolyzed wheat protein or partially hydrolyzed rice protein, are effective in repairing or mitigating the effects of dental erosion, promoting dental remineralization, and enhancing the anti-cavity effects of fluoride.

For example, in one embodiment, partially hydrolyzed plant proteins comprising oligo and polypeptide molecules having a molecular weight distribution of about 500D to about 10000 D, e.g. 1000D to 5000 D are prepared for formulation with ingredients of a suitable orally acceptable carrier, by diluting in buffer, e.g., a phospate buffer such as Na2HPO4 buffer (1.5 mM) and $CaCl_2$ (2.5 mM), to provide a buffered solution having a desired pH, e.g. pH 6-8, e.g., pH 7-8, e.g., about pH 7.5, filtering and centrifuging the solution to obtain a filtrat comprising the partially hydrolyzed plant protein. A biocide (for example cetylpyridinium chloride, e.g., at 0.1%) and/or fluoride may be added to the filtrate. The partially hydrolyzed plant protein may then be combined with components of an orally acceptable carrier, for example a toothpaste or mouthwash base, to provide an oral care composition for repairing or mitigating the effects of dental erosion, promoting dental remineralization, and enhancing the anti-cavity effects of fluoride.

The present invention refers to an oral care composition comprising (Composition 1):

a) partially hydrolyzed plant protein, comprising oligo- and polypeptide molecules having a molecular weight distribution of about 500 to about 10000 D;

b) an orally acceptable carrier,

for use in a method of repairing or inhibiting dental erosion, promoting dental remineralization, and/or enhancing the anti-cavity effects of fluoride comprising applying to the teeth said oral care composition, wherein the partially hydrolyzed plant protein is present in the composition in an amount of from 0.1 weight % to 3 weight % by total weight of the composition and wherein the partially hydrolyzed plant protein is selected from partially hydrolyzed wheat protein, partially hydrolyzed rice protein and combinations thereof. For example, the present invention refers to:

1.1. Composition 1 according to the claims wherein the partially hydrolyzed plant protein is partially hydrolyzed wheat protein or hydrolyzed rice protein.

1.2. Composition 1 according to the claims wherein the partially hydrolyzed plant protein is obtained from wheat, rice, or combinations thereof.

1.3. Any foregoing Composition according to the claims wherein the partially hydrolyzed plant protein is partially hydrolyzed wheat protein which is substantially free of gluten.

1.4. Any foregoing Composition according to the claims wherein the partially hydrolyzed plant protein comprises fluoride.

1.5. Any foregoing Composition according to the claims wherein the partially hydrolyzed plant protein has been adjusted to a desired pH, e.g., pH 6-8, e.g., pH 7-8, e.g, by using a phosphate buffer.

1.6. Any foregoing Composition according to the claims wherein the partially hydrolyzed plant protein comprises a biocide, e.g., cetylpyridinium chloride (CPC) at an effective concentration, e.g., 0.1% by weight of the filtrate.

1.7. Any foregoing Composition according to the claims wherein the partially hydrolyzed plant protein is present in the composition in an amount of from 0.1 weight % to 3 weight % by total weight of the composition, e.g., 0.2 weight% to 2 weight%, e.g., 0.2 weight %, 1 weight %, 1.5 weight % or 2 weight percent by total weight of the composition.

1.8. Any foregoing composition wherein the Composition according to the claims which comprises an effective amount of fluoride.

1.9. Any foregoing Composition according to the claims which comprises an amount of fluoride of 100 ppm to 2500 ppm, for example, 250 ppm to 750 ppm, for example about 500 ppm fluoride.

1.10. Any foregoing Composition according to the claims which comprises an amount of fluoride of 2500 ppm to 5000 ppm.

1.11. Any foregoing Composition according to the claims comprising an orally acceptable zinc salt or oxide, for example selected from zinc oxide, zinc citrate, zinc lactate, zinc phosphate, zinc acetate, zinc chloride, zinc complexes with amino acids, and mixtures of any of the foregoing, for example wherein the amount of zinc is from 0.1 weight % to 3 weight %, e.g., about 1 to about 2 weight %, calculated by weight of zinc ion.

1.12. Any foregoing Composition according to the claims comprising an orally acceptable stannous salt, for example $SnF_2$ or $SnCl_2$.

1.13. Any foregoing Composition according to the claims wherein the composition is in a form selected from a mouthrinse, a toothpaste, a tooth gel, a tooth powder, a non-abrasive gel, a mousse, a foam, a mouth spray, chewing gum, and a tablet, for example dentifrice, e.g., a toothpaste or mouthrinse.

1.14. Any foregoing Composition according to the claims, wherein the composition further comprises one or more agents selected from: abrasives, pH modifying agents, surfactants, foam modulators, thickening agents, viscosity modifiers, humectants, anti-calculus or tartar control agents, sweeteners, flavorants and colorants.

1.15. Any foregoing Composition according to the claims wherein the composition is a toothpaste.

1.16. Any foregoing Composition according to the claims comprising one or more soluble phosphate salts, for example wherein by "soluble phosphate salts" is meant an orally acceptable phosphate salt having a solubility in water of at least 1 g/100ml at 25°C; for example wherein the one or more soluble phosphate salts are sodium and/or potassium salts of pyrophosphates and/or polyphosphates, e.g., tripolyphosphates; e.g., wherein the one or more soluble phosphate salts comprise tetrasodium pyrophosphate (TSPP), sodium tripolyphosphate (STPP) or a combination of TSPP and STPP; for example, whereon the one or more soluble phosphate salts are present in an amount of 1-20%, e.g., 2-8%, e.g., ca. 5%, by weight of the composition.

1.17. Any foregoing Composition according to the claims wherein the fluoride is provided by a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.

1.18. Any foregoing Composition according to the claims which is a dentifrice comprising a humectant, e.g., selected from glycerin, sorbitol, propylene glycol, polyethylene glycol, xylitol, and mixtures thereof, e.g. comprising at least 30%, e.g., 40-50% glycerin, by weight of the composition.

1.19. Any foregoing Composition according to the claims which is a dentifrice comprising one or more surfactants, e.g., selected from anionic, cationic, zwitterionic, and nonionic surfactants, and mixtures thereof, e.g., wherein the dentifrice base comprises an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS) by weight of the composition.

1.20. Any foregoing Composition according to the claims which is a dentifrice comprising a zwitterionic surfactant, for example a betaine surfactant, for example cocamidopropylbetaine, e.g. in an amount of from 0.1% to 4.5% by weight, e.g. 0.5-2% cocamidopropylbetaine by weight of the composition

1.21. Any foregoing Composition according to the claims which is a dentifrice comprising a viscosity modifying amount of one or more of polysaccharide gums, for example xanthan gum or carrageenan, silica thickener, and combinations thereof.

1.22. Any foregoing Composition according to the claims which is a dentifrice comprising gum strips or fragments.

1.23. Any foregoing Composition according to the claims which is a chewing gum.

1.24. Any foregoing Composition according to the claims comprising flavoring, fragrance and/or coloring.

1.25. Any foregoing Composition according to the claims which is a dentifrice comprising an effective amount of one or more antibacterial agents, for example comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan or cetylpyridinium chloride.

1.26. Any foregoing Composition according to the claims which is a dentifrice comprising a whitening agent, e.g., a selected from the group consisting of peroxides, metal chlorites, perborates, percarbonates, peroxyacids, hypochlorites, and combinations thereof; e.g. hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate).

1.27. Any foregoing Composition according to the claims which is a dentifrice comprising an agent that interferes with or prevents bacterial attachment, e.g., solbrol or chitosan.

1.28. Any foregoing Composition according to the claims which is a dentifrice comprising a soluble calcium salt, e.g., selected from calcium sulfate, calcium chloride, calcium nitrate, calcium acetate, calcium lactate, and combinations thereof.

1.29. Any foregoing Composition according to the claims which is a dentifrice comprising a physiologically or orally acceptable potassium salt, e.g., potassium nitrate or potassium chloride, in an amount effective to reduce dentinal sensitivity.

1.30. Any foregoing Composition according to the claims which is a dentifrice comprising an anionic polymer, e.g., a synthetic anionic polymeric polycarboxylate, e.g., wherein the anionic polymer is selected from 1:4 to 4:1 copolymers of maleic anhydride or acid with another polymerizable ethylenically unsaturated monomer; e.g., wherein the anionic polymer is a methyl vinyl ether/maleic anhydride (PVM/MA) copolymer having an average molecular weight (M.W.) of 30,000 to 1,000,000, e.g. 300,000 to 800,000, e.g., wherein the anionic polymer is 1-5%, e.g., 2%, of the weight of the composition.

1.31. Any foregoing Composition according to the claims which is a dentifrice comprising a breath freshener, fragrance or flavoring.

1.32. Any of the foregoing Compositions according to the claims, wherein the pH of the composition is approximately neutral, e.g., pH 6-8, e.g., about pH 7.

1.33. Any of the forgoing compositions according to the claims for use in a method of repairing or inhibiting dental erosion, promoting dental remineralization, and/or enhancing the anti-cavity effects of fluoride comprising applying to the teeth said oral care composition.

1.34. Any foregoing Composition according to the claims for use in inhibiting or repairing dental enamel erosion.

1.35. Any foregoing composition according to the claims for use in promoting remineralization of dental enamel.

1.36. Any foregoing composition according to the claims for use in enhancing the anticavity effects of fluoride.

Disclosed is a dentifrice comprising

a) a) partially hydrolyzed plant protein, comprising oligo- and polypeptide molecules having a molecular weight wherein at least 95% of the plant protein is from about 500 to about 10000 D, e.g. about 1000 to about 5000 D; e.g. having a molecular weight distribution as follows: about 65% < 3500D, about 84-92% < 6000D, about 0.9-3.6% > 9000D, and about 0.1-0.6% > 13000D; for example wherein the amount of hydrolyzed wheat protein is 0.1 weight % to 2 weight %, for example about 0.2% hydrolyzed wheat protein;
b) optionally an effective amount of fluoride; for example wherein fluoride is present in an amount of 100 ppm to 1500 ppm, for example, about 500 ppm; and
c) an orally acceptable carrier.

Further disclosed is a mouthwash (Composition 1A) comprising:

a) partially hydrolyzed plant protein, comprising oligo- and polypeptide molecules having a molecular weight wherein at least 95% of the oligo- and polypeptide molecules are from about 500 to about 10000 D, e.g. about 1000 to about 5000 D; e.g. having a molecular weight distribution as follows: about 65% < 3500D, about 84-92% < 6000D, about 0.9-3.6% > 9000D, and about 0.1-0.6% > 13000D;
b) an orally acceptable carrier,

wherein the partially hydrolyzed plant protein is present in the composition in an amount of from 0.01 weight % to 3 weight % by total weight of the composition.
e.g., a mouthwash according to any of Composition 1, *et seq.,* e.g.,

1A.1. Composition 1A wherein the partially hydrolyzed plant protein is obtained from grains of the family *Poaceae,* e.g., is partially hydrolyzed maize (corn), wheat, rice, barley, oat, or millet protein.
1A.2. Composition 1A.1 wherein the partially hydrolyzed plant protein is partially hydrolyzed wheat protein or hydrolyzed rice protein.
1A.3. Any foregoing Composition 1A wherein the partially hydrolyzed plant protein is partially hydrolyzed wheat protein which is substantially free of gluten.
1A.4. Any foregoing Composition 1A, wherein the partially hydrolyzed plant protein is obtained from wheat, rice, almond, potato, pea, soya or combinations thereof.
1A.5. Any foregoing Composition 1A wherein the partially hydrolyzed plant protein comprises fluoride.
1A.6. Any foregoing Composition 1A wherein the partially hydrolyzed plant protein has been adjusted to a desired pH, e.g., pH 6-8, e.g., pH 7-8, e,g, by using a phosphate buffer.
1A.7. Any foregoing Composition 1A wherein the partially hydrolyzed plant protein comprises a biocide, e.g., cetylpyridinium chloride (CPC) at an effective concentration, e.g., 0.01 - 0.1% by weight of the filtrate.
1A.8. Any foregoing Composition 1A wherein the partially hydrolyzed plant protein is present in the composition in an amount of from 0.1 weight % to 3 weight % by total weight of the composition, e.g., 0.2 weight% to 2 weight%, e.g., about 0.2 weight %, by total weight of the composition.
1A.9. Any foregoing Composition 1A wherein the Composition comprises an effective amount of fluoride, e.g., a synergistically effective amount of fluoride, e.g., an amount of fluoride which in combination with the partially hydrolyzed plant protein, is effective to reduce or inhibit demineralization and/or enhance remineralization of the tooth enamel.
1A.10. Any foregoing Composition 1A comprising an effective amount of fluoride from a fluoride ion source selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.
1A.11. Any foregoing Composition 1A comprising a humectant, e.g., selected from glycerin, sorbitol, propylene glycol, polyethylene glycol, xylitol, and mixtures thereof, e.g., in an amount of 10-25% by weight, e.g. 18-20%,
1A.12. Any foregoing Composition 1A comprising an anionic surfactant, for example sodium lauryl sulfate or sodium lauryl ether sulfate or mixtures thereof, e.g., in an amount of 0.01-0.5%, e.g., about 0.05% by weight.
1A.13. Any foregoing Composition 1A comprising a nonionic surfactant, for example (i) a copolymer of propylene glycol and ethylene glycol, for example a triblock copolymer consisting of a central hydrophobic block of polypropylene glycol flanked by two hydrophilic blocks of polyethylene glycol, e.g., a poloxamer, e,g, poloxamer 188, poloxamer 338, poloxamer 407, or mixtures thereof, e.g., in an amount of 0.1-2% by weight, e.g., about 0.5%, and/or (ii) a polysorbate, for example, polysorbate 20, e.g., in an amount of 0.1-1% by weight, e.g., about 0.5%, and/or (iii) a polyethoxylated vegetable oil, e.g., polyoxyl 40 hydrogenated castor oil, e.g., in an amount of 0.05-0.5% by weight, e.g., about 0.1%.

1A.14. Any foregoing Composition 1A comprising a breath freshener, fragrance or flavoring.

1A.15. Any foregoing Composition 1A, wherein the pH of the composition is approximately neutral, e.g., pH 6-8, e.g., about pH 7.

1 A.16. Any foregoing Composition 1A comprising partially hydrolyzed wheat protein, e.g. in an amount of from 0.05-1% by weight, e.g., about 0.2%, and a fluoride ion source providing 200 to 1000 ppm fluoride, e.g., about 225 ppm or about 500 ppm fluoride.

1A.17. Any foregoing Composition 1A which is substantially free of ethanol, e.g. having less than 0.5% ethanol by weight.

1A.18. Any foregoing Composition 1A which is palatable and safe for use as a mouthwash.

1A.19. Any foregoing Composition 1A comprising

  a) partially hydrolyzed wheat protein, e.g. in an amount of from 0.05-1% by weight, e.g., about 0.2%,
  b) a fluoride ion source providing 200 to 1000 ppm fluoride, e.g., about 225 ppm or about 500 ppm fluoride, and
  c) cetylpyridinium chloride, e.g. in an amount of 0.01%-0.1% by weight, e.g., about 0.04%.

1A.20. Any foregoing Composition 1A which is a mouthwash comprising

  a) partially hydrolyzed wheat protein, e.g. in an amount of from 0.05-1% by weight, e.g., about 0.2%,
  b) a fluoride ion source, e.g., providing 200 to 1000 ppm fluoride, e.g., about 225 ppm or about 500 ppm fluoride,
  c) cetylpyridinium chloride, e.g. in an amount of 0.01%-0.1% by weight, e.g., about 0.04%,
  d) nonionic surfactant, e.g. poloxamer, e.g. in an amount of 0.1-1% by weight, e.g., about 0.5%,
  e) humectant, e.g. comprising sorbitol, glycerin, propylene glycol, and/or mixtures of any two or more of those, in an amount of 10-25% by weight, e.g. 18-20%,
  f) sweetener and flavorings,
  g) optionally a preservative, e.g. potassium sorbate, e.g., in an amount of 0.01-0.1% by weight, e.g., about 0.05%, and
  h) water, e.g., in an amount of 75-85% by weight.

1A.21. Any foregoing Composition 1A for use to reduce and inhibit acid erosion, clean the teeth, reduce bacterially-generated biofilm and plaque, reduce gingivitis, inhibit tooth decay and formation of cavities, and reduce dentinal hypersensitivity.

1A.22. Any foregoing Composition 1A for use in reducing, inhibiting or repairing dental enamel erosion.

1A.23. Any foregoing Composition 1A for use in promoting remineralization of dental enamel.

1A.24. Any foregoing Composition 1A for use in enhancing the anti-cavity and repair effects of fluoride.

In one aspect, the disclosure provides a composition according to the claims for use in repairing or inhibiting dental erosion, promoting remineralization , and/or enhancing the anticavity effects of fluoride; for example for use in any of the following methods according to the claims.

Disclosed is a method (Method 1) of repairing or inhibiting dental erosion, promoting dental remineralization , and/or enhancing the anti-cavity effects of fluoride comprising applying to the teeth a composition, e.g., any of Composition 1 according to the claims, for example an oral care composition comprising:

  a) partially hydrolyzed plant protein, comprising oligo- and polypeptide molecules having a molecular weight wherein the plant protein is from about 500 to about 10000 D, e.g. about 1000 to about 5000 D; e.g. having a molecular weight distribution as follows: about 65% < 3500D, about 84-92% < 6000D, about 0.9-3.6% > 9000D, and about 0.1-0.6% > 13000D; and
  b) an orally acceptable carrier,

wherein the partially hydrolyzed plant protein is present in the composition in an amount of from 0.1 weight % to 3 weight % by total weight of the composition. Further disclosed is:

1.1. Method 1 wherein the partially hydrolyzed plant protein is obtained from wheat, rice, almond, potato, pea, soya or combinations thereof; for example from grains of the family *Poaceae*, e.g., is partially hydrolyzed maize (corn), wheat, rice, barley, oat, or millet protein.

1.2. Method 1.1 wherein the partially hydrolyzed plant protein is partially hydrolyzed wheat protein or hydrolyzed rice protein.

1.3. Method 1 wherein the partially hydrolyzed plant protein is obtained from almond, potato or soya.

1.4. Any foregoing Method wherein the partially hydrolyzed plant protein comprises fluoride.

1.5. Any foregoing Method wherein the partially hydrolyzed plant protein has been adjusted to the desired pH, e.g., pH 6-8, for example pH 7-8, e.g., using a phosphate buffer.

1.6. Any foregoing Method wherein the partially hydrolyzed plant protein comprises a biocide, e.g., cetylpyridinium chloride (CPC) at an effective concentration, e.g., 0.1% by weight of the filtrate.

1.7. Any foregoing Method wherein the partially hydrolyzed plant protein is present in the composition in an amount of from 0.1 weight % to 3 weight % by total weight of the composition, e.g., 0.2 weight% to 2 weight%, e.g., about 0.2 weight %, about 1 weight %, about 1.5 weight % or about 2 weight percent by total weight of the composition.

1.8. Any foregoing composition wherein the Composition comprises an effective amount of fluoride.

1.9. Any foregoing Method wherein the amount of fluoride is 100 ppm to 1000 ppm, for example, about 500 ppm fluoride.

1.10. Any foregoing Method wherein the composition is in a form selected from a mouthrinse, a toothpaste, a tooth gel, a tooth powder, a non-abrasive gel, a mousse, a foam, a mouth spray, and a tablet.

1.11. Any foregoing Method, wherein the composition further comprises one or more agents selected from: abrasives, pH modifying agents, surfactants, foam modulators, thickening agents, viscosity modifiers, humectants, anti-calculus or tartar control agents, sweeteners, flavorants and colorants.

1.12. Any foregoing Method wherein the composition is a dentifrice, e.g., a toothpaste.

1.13. Any foregoing Method wherein the composition is selected from any of Compositions 1, *et seq., supra.*

1.14. Any foregoing Method which is a method for reducing, inhibiting or repairing dental erosion, for example erosion of the enamel, for example wherein the composition is applied to the teeth of a patient having been identified as having dental erosion or being at elevated risk of having dental erosion.

1.15. Any foregoing Method which is a method for promoting dental remineralization, for example remineralization of the enamel, for example wherein the composition is applied to the teeth of a patient having been identified as having demineralization.

1.16. Any foregoing Method which is a method for enhancing the anti-cavity effects of fluoride, for example wherein the composition is applied to the teeth of a patient having been identified as having early signs of tooth decay, for example early enamel caries, or as having active decay, or as being at elevated risk of tooth decay.

1.17. Method 1.16 wherein the composition comprises an effective amount of fluoride and/or wherein the method further comprises administration of an oral care product comprising an effective amount of fluoride mouth rinse or toothpaste comprising an effective amount of fluoride.

1.18. Any foregoing Method wherein the composition is selected from any of Composition 1, et seq.

1.19. Any foregoing Method wherein the composition is selected from any of Composition 1A, et seq.

Further disclosed is the use of a partially hydrolyzed plant protein, for example a partially hydrolyzed wheat protein or partially hydrolyzed rice protein, in the manufacture of an oral care composition, for example according to any of Compositions 1, *et seq.,* e.g., any of Compositions 1A, *et seq.,* for repairing or inhibiting dental erosion, promoting remineralization, and/or enhancing the anti-cavity effects of fluoride, e.g., in any of Methods 1, *et seq.*

Further disclosed is a method (Method 2) of making an oral care product, e.g. an oral care product useful for repairing or inhibiting dental erosion, promoting dental remineralization, and/or enhancing the anti-cavity effects of fluoride, e.g., a product according to any of Composition 1, et seq., e.g., any of Composition 1A, et seq., comprising

a) a partially hydrolyzed plant protein, comprising oligo- and polypeptide molecules having a molecular weight distribution of 500 to 10000 D, e.g. 1000 to 5000 D, adjusting the pH by dilution with an aqueous buffer solution, e.g., a phosphate buffer solution, e.g., to obtain a solution having the desired pH, e.g., pH 6-8, e.g., pH 7-8;

b) filtering and centrifuging the solution product of a) to obtain a filtrate comprising the partially hydrolyzed plant protein;

c) adding fluoride (e.g., in the form of an orally acceptable salt containing fluoride, for example sodium fluoride or sodium monofluorophosphate), and optionally a biocide (e.g., cetylpyridinium chloride at an effective amount, e.g., 0.01 to 1%, e.g., 0.1% by weight of the filtrate) to the filtrate product of b);

d) admixing the product of c) to components of an orally acceptable carrier to obtain an oral care composition comprising the partially hydrolyzed plant protein in an amount of from 0.01 weight % to 3 weight % by total weight of the composition.

For example, disclosed is:

2.1. Method 2 wherein the partially hydrolyzed plant protein is obtained from wheat, rice, almond, potato, pea, soya or combinations thereof, for example from grains of the family *Poaceae*, e.g., is partially hydrolyzed maize (corn), wheat, rice, barley, oat, or millet protein.

2.2. Method 2.1 wherein the partially hydrolyzed plant protein is partially hydrolyzed wheat protein or hydrolyzed rice protein.

2.3. Method 2 wherein the partially hydrolyzed plant protein is obtained from almond, potato or soya.

2.4. Any foregoing method wherein the composition is a composition according to any of Composition 1, et seq.

2.5. Any foregoing method wherein the composition is a composition according to any of Composition 1A, et seq.

For example, the disclosure provides an oral care composition comprising partially hydrolyzed plant protein, e.g., a composition according to any of Composition 1, et seq., for example any of Composition 1A, et seq., wherein the oral care composition is obtained or obtainable by the process of Method 2, et seq.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

## DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Hydrolyzed plant proteins

Hydrolyzed plant proteins are proteins from plants, for example, from edible plant parts, for example from wheat, rice, almond, potato, pea, soya or combinations thereof, e.g., from cereal grains such as maize, wheat, rice, barley, oats, and millet. The hydrolyzed plant proteins according to the claims are from wheat or rice or combinations thereof.

Hydrolyzed wheat protein is typically obtained by enzymatically hydrolyzing wheat proteins using endoproteases and exoproteases. Hydrolyzed wheat protein may also be obtained through acid or alkaline hydrolysis. Methods of preparing hydrolyzed wheat protein would be known to the person skilled in the art of protein chemistry. However, hydrolyzed wheat protein is also commercially available as Gluadin® W20, Gluadin W40 from BASF, and as Wheatpro® from IKEDA. Gluadin W20 is a partial hydrolysate obtained through enzymatic hydrolysis of wheat gluten. It contains at least 20.0 % of dry substance. Gluadin W40 is a partial hydrolysate obtained through enzymatic hydrolysis of wheat gluten. It contains at least 40.0 % of dry substance.

In another embodiment, the hydrolyzed plant protein is made from processed wheat protein which is free of gluten.

Hydrolyzed rice protein is typically obtained by enzymatically hydrolyzing rice protein using endoproteases and exoproteases. Hydrolyzed rice protein may also be obtained through acid or alkaline hydrolysis. Methods of preparing hydrolyzed rice protein would be known to the person skilled in the art of protein chemistry. However, hydrolyzed rice protein is also commercially available as Gluadin® R from BASF, and as Rice Pro-Tein BK-S® from TRI-K Industries

The hydrolyzed plant protein used in the compositions and methods herein is not *fully* hydrolyzed and thus is sometimes referred to as "partially hydrolyzed" to emphasize this point. By "partially hydrolyzed" it is meant that at least some, but not all, of the peptide bonds are hydrolyzed. Thus, the hydrolyzed plant protein typically comprises a mixture of amino acids and peptides of varying size. MW of relevant active ingredient is in the range of about 500 - about 10,000 D, for example about 1000 to about 5000 D.

The hydrolyzed plant protein according to the claims is present in the composition in an amount of from 0.1 weight % to 3 weight % by total weight of the composition. The hydrolyzed plant protein according to the claims is present in the composition in an amount of from 0.1 weight % to 3 weight %, or from 0.1 weight % to 2 weight %, or from 0.1 weight % to 1 weight % by total weight of the composition. In other embodiments, the hydrolyzed plant protein according to the claims is present in the composition in an amount of from 0.1 weight % to 0.5 by total weight of the composition. In further embodiments, the hydrolyzed plant protein according to the claims is present in the composition in an amount of from 0.5 weight % to 3 weight %, or from 0.5 weight % to 2 weight %, or from 0.5 weight % to 1 weight % by total weight of the composition. In still further embodiments, the hydrolyzed plant protein according to the claims is present in the composition in an amount of from 1 weight % to 3 weight %, or from 1 weight % to 2 weight % by total weight of the composition.

In one arrangement, the compositions of the present invention comprise both hydrolyzed wheat protein and hydrolyzed rice protein. In this arrangement, the hydrolyzed wheat protein and the hydrolyzed rice protein may be present in the composition in the amounts defined above. The total amount of hydrolyzed wheat protein and hydrolyzed rice protein in the composition is from 0.1 weight % to 3 weight %, or from 0.1 weight % to 2 weight %, or from 0.1 weight % to 1 weight %, or from 0.1 weight % to 0.5 weight % by total weight of the composition. In some embodiments, the total amount of hydrolyzed wheat protein and hydrolyzed rice protein in the composition is from 1 weight % to 3 weight %, or from 1 weight % to 2 weight %, by total weight of the composition.

Orally acceptable carrier and optional ingredients

The expression "orally acceptable carrier" as used herein denotes a carrier made from materials that are safe and acceptable for oral use in the amounts and concentrations intended, for example materials as would be found in conventional toothpaste and mouthwash. Such materials include water or other solvents that may contain a humectant such as glycerin, sorbitol, xylitol and the like. In some aspects, the term "orally acceptable carrier" encompasses all of the components of the oral care composition except for the hydrolyzed plant protein and the fluoride. In other aspects, the term refers to inert or inactive ingredients that serve to deliver the hydrolyzed plant protein, and/or any other functional ingredients, to the oral cavity.

Orally acceptable carriers for use in the invention include conventional and known carriers used in making mouth rinses or mouthwashes, toothpastes, tooth gels, tooth powder, lozenges, gums, beads, edible strips, tablets and the like. Carriers should be selected for compatibility with each other and with other ingredients of the composition.

The following non-limiting examples are provided. In a toothpaste composition, the carrier is typically a water/humectant system that provides a major fraction by weight of the composition. Alternatively, the carrier component of a toothpaste composition may comprise water, one or more humectants, and other functional components other than the hydrolyzed wheat protein or hydrolyzed rice protein. In a mouth rinse or a mouthwash formulation, the carrier is typically a water/alcohol liquid mixture in which the hydrolyzed wheat protein or hydrolyzed rice protein is dissolved or dispersed. In a dissolvable lozenge, the carrier typically comprises a solid matrix material that dissolves slowly in the oral cavity. In chewing gums, the carrier typically comprises a gum base, while in an edible strip, the carrier typically comprises one or more film forming polymers.

The oral care compositions provided herein may further comprise one or more additional ingredients selected from abrasives, pH modifying agents, surfactants, foam modulators, thickening agents, viscosity modifiers, humectants, anti-calculus or tartar control agents, sweeteners, flavorants, colorants and preservatives. These ingredients may also be regarded as carrier materials. Non-limiting examples are provided below.

In one embodiment a composition of the invention comprises at least one abrasive, useful, for example, as a polishing agent. Any orally acceptable abrasive can be used, but the type, fineness (particle size) and amount of abrasive should be selected so that tooth enamel is not excessively abraded during normal use of the composition. Suitable abrasives include, without limitation, silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, [beta]-calcium pyrophosphate, tricalcium phosphate, calcium polymetaphosphate and insoluble sodium polymetaphosphate. One or more abrasives are optionally present in the oral care compositions of the present invention in an amount of 1 weight % to 5 weight % by total weight of the composition. The average particle size of an abrasive, if present, is generally 0.1 to 30$\mu$m, and preferably, 5 to 15$\mu$m.

In a further embodiment an oral care composition of the invention comprises at least one bicarbonate salt, useful, for example, to impart a "clean feel" to teeth and gums due to effervescence and release of carbon dioxide. Any orally acceptable bicarbonate can be used, including, without limitation, alkali metal bicarbonates such as sodium and potassium bicarbonates, ammonium bicarbonate and the like. One or more bicarbonate salts are optionally present in a total amount of 1 weight % to 10% by weight of the composition.

In a still further embodiment, an oral care composition of the invention comprises at least one pH modifying agent. Such agents include acidifying agents to lower pH, basifying agents to raise pH and buffering agents to control pH within a desired range. For example, one or more compounds selected from acidifying, basifying and buffering agents can be included to provide a pH of 2 to 10, or in various illustrative embodiments a pH of 2 to 8, 3 to 9, 4 to 8, 5 to 7, 6 to 10, or 7 to 9. Any orally acceptable pH modifying agent can be used, including, without limitation, carboxylic, phosphoric and sulfonic acids, acid salts (for example, monosodium citrate, disodium citrate, monosodium malate), alkali metal hydroxides such as sodium hydroxide, carbonates such as sodium carbonate, bicarbonates, borates, silicates, phosphates (for example, monosodium phosphate, trisodium phosphate, pyrophosphate salts) imidazole and the like. One or more pH modifying agents are optionally present in a total amount effective to maintain the composition in an orally acceptable pH range.

In a still further embodiment a composition of the invention comprises at least one surfactant, useful, for example, to provide enhanced stability to the composition and the components contained therein, to aid in cleaning a dental surface through detergent action, and to provide foam upon agitation (for example, during brushing with a dentifrice composition of the invention). Any orally acceptable surfactant, including those which are anionic, nonionic or amphoteric, can be used. Suitable anionic surfactants include, without limitation, water-soluble salts of $C_{8-20}$ alkyl sulfates, sulfonated monoglycerides of $C_{8-20}$ fatty acids, sarcosinates, taurates and the like. Suitable nonionic surfactants include, without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants , without limitation, derivatives of $C_{8-20}$ aliphatic secondary and tertiary amines having an anionic group such as carboxylate, sulfate, sulfonate, phosphate or phosphonate. A suitable example is cocoamidopropyl betaine. One or more surfactants are optionally present in a total amount of 0.01 weight % to 10 weight %, for example, from 0.05 weight % to 5 weight % or from 0.1 weight % to 2 weight % by total weight of the composition.

In a still further embodiment, an oral care composition of the invention comprises at least one foam modulator, useful, for example, to increase the amount, thickness or stability of foam generated by the composition upon agitation. Any orally acceptable foam modulator can be used including, without limitation, polyethylene glycols (PEGs). One or more PEGs are optionally present in a total amount of from 0.1 weight % to 10 weight by total weight of the composition.

In a still further embodiment, an oral care composition of the invention comprises at least one thickening agent, useful, for example, to impart a desired consistency and/or mouth feel to the composition. Any orally acceptable thickening agent can be used including, without limitation, carbomers (carboxyvinyl polymers), carrageenans, cellulosic polymers such as hydroxyethylcellulose, carboxymethylcellulose (CMC) and salts thereof, natural gums such as karaya, xanthan, gum arabic and tragacanth, colloidal magnesium aluminum silicate, colloidal silica and the like. One or more thickening agents are optionally present in a total amount of 0.01 weight % to 15 weight %, by total weight of the composition.

In a still further embodiment a composition of the invention comprises at least one viscosity modifier, useful, for example, to inhibit settling or separation of ingredients or to promote redispersion of ingredients upon agitation of a liquid composition. Any orally acceptable viscosity modifier can be used including, without limitation, mineral oil, petrolatum, clays, silica and the like. One or more viscosity modifiers are optionally present in a total amount of 0.01 weight % to 10 weight %, by total weight of the composition.

In a still further embodiment, an oral care composition of the invention comprises at least one humectant which may be used to prevent hardening of a toothpaste upon exposure to air, or in the case of a mouthwash, to provide improved moisturizing and mouthfeel and enhance the miscibility of poorly soluble components such a flavoring oils. Any orally acceptable humectant can be used, including, without limitation, polyhydric alcohols such as glycerin, sorbitol, xylitol or low molecular weight PEGs. Most humectants also function as sweeteners. One or more humectants are optionally present in a total amount of 1 weight % to 50 weight % by total weight of the composition.

In a still further embodiment, an oral care composition of the invention comprises at least one sweetener which enhances taste of the composition. Any orally acceptable natural or artificial sweetener can be used including, without limitation, dextrose, sucrose, maltose, dextrin, mannose, xylose, ribose, fructose, levulose, galactose, corn syrup, partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount of 0.005 weight % to 5 weight % by total weight of the composition.

In a still further embodiment, an oral care composition of the invention comprises at least one flavorant which enhances the taste of the composition. Any orally acceptable natural or synthetic flavorant can be used including, without limitation, vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences, and the like. Also encompassed within flavorants are ingredients that provide fragrance and/or other sensory effects in the mouth, including cooling or warming effects. Such ingredients illustratively include menthol, menthyl acetate, menthyl lactate, camphor, eucalyptus oil, eucalyptol, eugenol, cassia, oxanone, $\alpha$-irisone, thymol, linalool, benzaldehyde, cinnamaldehyde, N-ethyl- *p*-menthan-3-carboxamine, N,2,3-trimethyl-2-isopropylbutanamide, 3 -( 1 -menthoxy)-propane- 1 ,2-diol, cinnamaldehyde glycerol acetal (CGA), menthone glycerol acetal (MGA) and the like. One or more flavorants are optionally present in a total amount of 0.01 weight % to 5 weight %, by total weight of the composition.

In a still further embodiment, an oral care composition of the invention comprises at least one colorant. A colorant can serve a number of functions. These include providing a white or lightcolored coating on a dental surface, indicating locations on a dental surface that have been effectively contacted by the composition, and/or modifying the appearance of the composition to enhance attractiveness to the consumer. Any orally acceptable colorant can be used including, without limitation, talc, mica, magnesium carbonate, calcium carbonate, magnesium silicate, magnesium aluminum silicate, silica, titanium dioxide, zinc oxide, iron oxide, ferric ammonium ferrocyanide, manganese violet, titaniated mica, bismuth oxychloride and the like. One or more colorants are optionally present in a total amount of 0.001 weight % to 20 weight % by total weight of the composition.

In a still further embodiment, an oral care composition of the invention comprises a preservative. The preservative may be selected from parabens, potassium sorbate, benzyl alcohol, phenoxyethanol, polyaminopropryl biguanide, caprylic acid, sodium benzoate and cetylpyridinium chloride. In some embodiments, the preservative is present at a concentration of from about 0.001 to about 1 weight %, by total weight of the composition.

The following examples illustrate compositions of the invention and their uses. The exemplified compositions are illustrative and do not limit the scope of the invention.

EXAMPLES

Example 1 - Surface Roughness

Bovine teeth are cut, ground and polished to obtain enamel blocks having approximate dimensions of 3mm x 3mm x 2mm. The thickness of the enamel is approximately 1 to 2mm, and the thickness of dentin is approximately 1mm. All measurements are taken on the enamel surface.

The surface roughness of enamel blocks is measured before and after acid etching. Acid etching is achieved by placing the enamel blocks in 1% citric acid solution (pH 3.8) until the surface roughness (Sq) reaches 100 - 200. This Sq value is recorded as the surface roughness of etched enamel. (Sq is a standard measurement defined in ISO 25178 basically as the root mean square height relative to the arithmetical mean height of the surface being measured, so it corresponds to the mean of all absolute distances of the profile from the center plane within the measuring area, such that if it is higher, it means that that there are more pronounced peaks and valleys on the surface, i.e., the surface is rougher, and if it is lower, the surface is smoother.) A hydrolyzed wheat protein preparation is made using GLUADIN W20 from BASF. The hydrolysate is diluted to the final concentration using a $Na_2HPO_4$ buffer (1.5 mM) and $CaCl_2$ (2.5 mM). After neutralizing to a pH of 7.5, the solution is filtered and centrifuged. Acid-etched enamel blocks are subsequently incubated with a solution comprising the treated hydrolyzed wheat protein at a concentration of 0.5 weight % (2ml solution per block) for 60 minutes.

After the 60 minute incubation period, the enamel blocks are incubated in artificial saliva (AS) solution (0.4g NaCl, 0.4g KCl, 0.8g $CaCl_2$, 0.69g $NaH_2PO_4$, 1g urea, 1 liter distilled water; pH 7 (adjusted using 1M NaOH)) for 22 hours. The enamel blocks are then treated a second time with the hydrolyzed protein solution, and incubated again with artificial saliva for 22 hours. The blocks are then rinsed with deionized (DI) water and air-dried prior to measuring their surface roughness. Enamel blocks treated with 0.5% PBS are used as a negative control for the experiment. The results are illustrated in Table 1. The % remineralization represents the % reduction in final surface roughness (Sq) relative to surface roughness of etched enamel (before treatment and artificial saliva incubation):

$$\% \text{ remineralization} = \frac{(Sq_{Etched} - Sq_{Repaired}) \times 100}{(Sq_{Etched} - Sq_{Baseline})}$$

Table 1 - Results of surface roughness assay

| Test solution | Mean % remineralization | S.D. |
|---|---|---|
| 0.5% Hydrolyzed wheat protein | 14.58% | 5.43% |
| PBS | -15.76% | 14.62% |

As indicated in Table 1, hydrolyzed wheat protein is effective in reducing the surface roughness of acid-etched enamel blocks at a concentration of 0.5 weight %.

Example 2 - Nanoindentation

Enamel blocks are prepared as described in Example 1. Acid-etching is achieved by placing the enamel blocks in 1% citric acid solution (pH 3.8) for 15 minutes. The nanohardness and Young's modulus at 500nm depth are measured prior to and after etching. Etched enamel blocks are incubated with a solution of hydrolyzed wheat protein (GLUADIN W20), at a concentration of 0.5 weight %, for 30 minutes (2ml solution/block), followed by an incubation with AS solution as described in Example 1, for 22 to 24 hours. The hydrolyzed wheat protein and AS incubation steps are repeated two additional times, after which the enamel blocks are rinsed with DI water and air-dried. The nanohardness and Young's modulus of the treated enamel blocks are measured at 500nm depth to assess the enamel repair effects of the hydrolyzed wheat protein. A solution of 500 ppm fluoride is used as a positive control for the experiment. The results are illustrated in Tables 2 and 3.

$$\% \text{ remineralization} = \frac{(\text{Repaired- Etched}) \times 100}{(\text{Sound baseline- Etched})}$$

Table 2 - Repair efficacy of hydrolyzed wheat protein - nanohardness

| Test | Nanohardness Repair at 500nm depth (%) |
|---|---|
| Hydrolyzed wheat protein | 38.06 |
| Fluoride (control) | 31.12 |

Table 3 - Repair efficacy of hydrolyzed wheat protein - Young's modulus

| Test | Young's Modulus Repair at 500nm depth (%) |
|---|---|
| Hydrolyzed wheat protein | 52.52 |
| Fluoride (control) | 48.50 |

As can be seen in Tables 2 and 3, hydrolyzed wheat protein is effective in repairing acid-eroded enamel.

Example 3 - Microhardness

Enamel blocks are prepared as described in Example 1. Microhardness is measured using a Micromet 6020 Micro-hardness Tester with a Knoop Diamond Indenter and a 50 g load (Buehler, Lake Bluff, IL, USA). Blocks with a Knoop hardness (KH) of at least 300 are selected. The blocks are etched by immersing in 30% phosphoric acid for 15 seconds. The KH on the left and right sides of the blocks are measured. Subsequently, the right side of the blocks are covered with tape prior to treating the blocks with 20mg/ml solution of hydrolyzed wheat protein (Gluadin W20 from BASF) or hydrolyzed rice protein (GLUADIN R from BASF), each neutralized as described in example 1, for two terms of 30 minutes. (The tape on the right hand side of the blocks prevents exposure of this side to the protein solution and serves as an internal control). The blocks are washed twice with DI water @ 5 minutes, 500 PRM between the two treatments and after the second treatment. Subsequently, the tape is removed and the blocks are incubated in AS solution (0.2 mM $MgCl_2$, 1mM $CaCl2.H_2O$, 20mM HEPES buffer, 4mM $KH_2PO_4$, 16 mM KCl, 4.5 mM $NH_4Cl$, 300 ppm NaF, 0.05 wt.% $NaN_3$, at pH 7 (adjusted with 1M NaOH)) for 7 days. After rinsing the enamel blocks with DI water and air-drying the rinsed blocks, microhardness is measured again. Surface microhardness regain (SMHL, Remin%) as a percent is calculated as

$$\% \text{remineralization} = \frac{(MH_{\text{Repaired}} - MH_{\text{Etched}}) \times 100}{(MH_{\text{Baseline}} - MH_{\text{Etched}})}$$

The results of the microhardness assay are illustrated in Table 4.

Table 4 - Results of microhardness assay

| Protein | % remineralization (mean) | S.D. |
|---|---|---|
| 2% Hydrolyzed wheat protein | 18.37% | 0.50% |
| 2% Hydrolyzed rice protein | 14.64% | 1.51% |
| Control buffer | 1.67% | 0.19% |

It can be seen from Table 4 that both hydrolyzed wheat protein and hydrolyzed rice protein are effective in remineralizing the enamel surface of acid-etched enamel blocks. Note that for all of the microhardness tests, the reminineralization percentage from the right/internal control side, which was immersed artificial salvia as well and had some repair from artificial salvia, is subtracted from the results, so that the data present the differential effect of the hydrolyzed protein.

This experiment is then extended to measure the effects of the hydrolyzed wheat and rice proteins in comparison and in combination with fluoride.

Two concentrations of hydrolysates (2 wt% and 0.2 wt%) mixed with fluoride (provided in the form of NaF) are tested, and the results show both 2 wt% and 0.2 wt% of wheat hydrolysate (Gluadin W20, BASF) and of rice hydrolysate (Gluadin R, BASF) can enhance fluoride repair. The hydrolysate is diluted using a $Na_2HPO_4$ buffer (1.5 mM) and $CaCl_2$ (2.5 mM). After neutralizing to a pH of 7.5, the solution is filtered and centrifuged. A biocide (CPC 0.1%) and fluoride (in varying amounts as described in table 5) are added to the filtrate, and the microhardness assay is carried out as described above.

Etched enamels treated with hydrolysate + fluoride have higher remineralization% compared to the samples treated with fluoride alone. Test are also run using alpha-fetoprotein (AFP) for comparison to the plant protein hydrolysates.

Remineralization% of 0.2% hydrolysate w/ different dose of fluoride are tested as well. Among the low concentration (0.2%) hydrolysate tests, 0.2 wt% wheat hydrolysate + 500 ppm fluoride shows the best result. There is only little improvement when the fluoride level was elevated to 900 ppm.

TABLE 5 - Hydrolysates enhance fluoride effect on enamel repair in microhardness assay;

| Sample | Remineralization % | S.D. |
|---|---|---|
| Buffer | 2.25% | 1.04% |
| Buffer + 250 ppm fluoride | 11.73% | 2.01% |
| Buffer + 500 ppm fluoride | 15.87% | 1.98% |
| 2% wheat hydrolysate | 18.08% | 1.12% |
| 2% wheat hydrolysate + 250 ppm fluoride | 30.84%* | 3.20% * |
| 0.2% wheat hydrolysate | 11.60% | 1.85% |
| 0.2% wheat hydrolysate + 250 ppm fluoride | 15.31% | 1.02% |
| 0.2% wheat hydrolysate + 500 ppm fluoride | 27.26% | 1.63% |
| 0.2% wheat hydrolysate + 900 ppm fluoride | 29.63% | 1.44% |
| 2% rice hydrolysate | 16.89% | 2.65% |
| 2% rice hydrolysate + 250 ppm fluoride | 28.20% * | 1.78% * |
| 0.2% rice hydrolysate | 9.30% | 1.79% |
| 0.2% rice hydrolysate + 250 ppm fluoride | 14.59% | 2.65% |
| 0.2% rice hydrolysate + 500 ppm fluoride | 25.45% | 1.24% |
| *Test carried out in a separate series of experiments; data pooled for comparative purposes. | | |

Example 4 - Enhancing anti-cavity effects of fluoride in a cavity model

Bovine enamel blocks prepared in accordance with Example 1 are prescreened, to select specimens with (i) scratch free enamel surface and (ii) KH > 300, measuring microhardness as described in Example 3. The selected enamel blocks are then the enamel blocks are pre-treated and subjected to 4 day cycling as described below, using DI water (negative control), 500 ppm F (positive control) and 0.2% wheat hydrolysate (Gluadin W20) + 500 ppm F (test material) prepared as described in the previous example. The microhardness of the enamel blocks is measured again as final ($MH_{final}$).

Pre-treatment: Enamel blocks are prepared as in Example 1. Each enamel block is sanitized in ethanol twice then washed in sterile DI water three times. Then each enamel block is pre-incubated in saliva supernatant at 4°C overnight to form pellicle on the enamel surface.

Day Two to Five - Dentifrice treatment, demineralization and remineralization: Unstimulated whole saliva is collected from donors, who refrain from eating and oral hygiene in the morning. The saliva is pooled and centrifuged at 8000 rpm for 10 minutes. The resulting salivary supernatant is decanted into a new sterile tube, and mixed with 1.5% Tryptic soy broth (TSB) at 2:1 ratio (2 parts saliva supernatant and 1 part TSB). The sediment is washed once using sterile DI water and centrifuged at 8000 rpm for 10 minutes. The water supernatant is removed. The washed sediment is then diluted in the salivary supernatant TSB mixture to an OD (610 nm) = 0.5. Sterile sucrose solution is added to a final concentration of 0.2% for first experiment and 0.4% for the second experiment (level of sucrose is increased in the second experiment to increase de-mineralization level). This mixture represents the reaction mixture. This reaction mixture serves as a demineralization and remineralization solution, since bacteria in saliva sediment will slowly metabolize sucrose to produce acid.

Enamel blocks are transferred into a 24 well plate with 2 ml of treatment solution (500 ppm F or 0.2% hydrolyzed wheat protein (Gluadin W20) + 500 ppm F) or negative control (DI water) in specific well. The plate is shaken at 100 rpm at room temperature for 10 minutes. After treatment, the enamel blocks are washed two times with sterile DI water. After washing, enamel blocks are transferred into a new 24 well plate containing 1 ml of the above reaction mixture per well and are incubated at 37°C for at least one hour. This treatment and incubation process is repeated for a total of four times during the day. After the 4th treatment the enamel blocks are left in reaction mixture incubated at 37°C overnight.

The above procedure is repeated for a total of four days (Day Two to Five). At the end of Day Five, enamel blocks are taken out of reaction mixture, washed and let dry on bench top.

Evaluation: Changes in the mechanical properties are assessed using a Micromet 6020 Micro-hardness Tester with a Knoop Diamond Indenter and a 25 g load (Buehler, Lake Bluff, IL, USA). Surface microhardness measurements are taken at baseline (confirmed sound enamel) and after treatment. Surface microhardness loss (SMHL, Demin%) as a percent was calculated as (1- ($Microhardness_{final}$/ $Microhardness_{sound}$))*100.

Examples 1-3 show that the hydrolyzed wheat protein can enhance the enamel repair property of fluoride. The results of this experiment show that hydrolyzed wheat protein plus fluoride provides higher anti-cavity efficacy than fluoride alone.

Two studies (with 0.2 % and 0.4% sucrose) on 0.2% wheat hydrolysate (Gluadin W20) + 500 ppm fluoride (provided as NaF) are completed using the above-described in vitro anticavity model.

The table below shows the results from the two studies. The first study is conducted using 0.2% sucrose for bacteria in saliva sediment to metabolize and produce acid. The second study is conducted with same procedure but using 0.4% sucrose. The level of sucrose in the second experiment is increased so as to product more acid, thus increase level of de-mineralization. Results from both of the studies show that 500 ppm F has better anticavity properties compared to DI water, but its anti-cavity property is still improved when mixed with 0.2% wheat hydrolysate.

Table 5 - Anti-cavity / demineralization protection of hydrolyzed wheat protein with fluoride

| Test (0.2% sucrose) | Demineralization percentage | S.D. |
|---|---|---|
| 0.2% hydrolyzed wheat protein + 500 ppm fluoride | 4.99% | 3.77% |
| 500 ppm fluoride | 8.36% | 4.05% |
| DI water | 33.08% | 2.18% |
| Test (0.4% sucrose) | Demineralization percentage | S.D. |
| 0.2% hydrolyzed wheat protein + 500 ppm fluoride | 13.69% | 6.58% |
| 500 ppm fluoride | 19.99% | 5.65% |
| DI water | 63.94% | 4.50% |

The results from both studies show that wheat hydrolysate + 500 ppm F has better the anti-cavity property (i.e., less demineralization) than 500 ppm F or DI water.

Example 5 - Remineralization and enhancing anticavity effects of fluoride - Clinical study

A treatment phase is completed ex vivo. Initial microhardness (MH) measurements are taken on sound, bovine enamel blocks ($MH_{Baseline}$). The blocks are then etched with 30% phosphoric acid until there was a 40% change in the microhardness reading from Baseline. The microhardness is recorded as etched (Equation 1, $MH_{Etched}$). Each etched enamel block is then immersed in a 2 ml solution of pre-treated 0.2% wheat hydrolysate (Gluadin W20) + 500 ppm F prepared as described above, or a 2 ml solution of 500 ppm F, for 30 minutes. After 30 minutes, the blocks are washed twice with DI water at 500 RPM for 5 minutes, and then dried with a paper towel. This treatment cycle is repeated. The blocks are then secured within the designated location on a retainer for placement in the oral cavity of a subject. Six enamel blocks are placed in each retainer. Pre-punched holes on the retainer allow saliva fluid to contact etched enamel and to provide minerals for remineralization. Each subject thus will have six enamel blocks in the retainer in the oral cavity during the study (3 are treated with the 0.2% wheat hydrolysate + 500 ppm F, and 3 are treated with 500 ppm F as control). The treated and control enamel blocks are randomized on the left or right side of the retainer. The retainers are then stored in a refrigerator until Day 1 of the study.

Twenty-six subjects are included in this randomized, blinded study. After a one week wash-out period where panelists brushed with anti-cavity toothpaste (1100 ppm fluoride), the subjects are given retainers containing the pre-treated enamel blocks. They were required to wear the retainer for 7 days (24hrs/day), while using the anti-cavity toothpaste in the morning and evening and an alcohol-free mouth rinse (100 ppm fluoride) after each meal (for a total of up to 3x/day). They are also required to rinse the retainer every night with water to avoid plaque accumulation. After 7 days, the enamel blocks are taken out of the appliance to take the final microhardness measurements ($MH_{Remineralized}$). Demineralization and remineralization are calculated as follows:

$$\% \text{ Demineralization} = \frac{(MH_{Baseline} - MH_{Etched}) \times 100}{(MH_{Baseline})} \qquad \text{equation 1}$$

$$\% \text{ Remineralization} = \frac{(MH_{Remineralized} - MH_{Etched}) \times 100}{(MH_{Baseline} - MH_{Etched})} \qquad \text{equation 2}$$

Two-2way ANOVA with and without a weight adjustment is used to analyze the data. A p-value of less than 0.05 is used to indicate statistical significance. Both statistical analyses showed significant differences between groups. The results below show that after 7 days, significantly greater remineralization is measured for the 0.2% wheat hydrolysate + 500 ppm F treatment groups compared to the 500 ppm F control group.

Table 6 - *In situ* clinical results

| Evaluation with weight adjustment | % Remin | Statistical Significance* | P value |
|---|---|---|---|
| 2 mg/ml (0.2%) wheat hydrolysate + 500 ppm fluoride | 56.2 | A | P =0.000 |
| Control 500 ppm fluoride | 27.6 | B | |
| *Means that do not share the same letter are significantly different. | | | |

The results of the analysis on the remineralization efficacy clinical study show that after two cycles of treatment *(ex vivo)* and 7 day remineralization *(in situ),* enamel blocks treated with 0.2% wheat hydrolysate + 500 ppm F exhibit significantly better remineralization when compared to a 500 ppm F control.

Example 6 - Mouthwash formulation

This example explores whether the surprising efficacy of the wheat hydrolysate in promoting remineralization is seen in a full mouthwash formula containing 0.2% wheat hydrolysate and 225 ppm F. Two different treatment methods are tested: one treatment (for 7 days) or repeated treatment (once/day for 7 days). The results show wheat hydrolysate keeps its remineralization benefit in both one treatment and repeated treatment; and wheat hydrolysate has better repair efficacy in repeated treatment.

A test mouthwash and a control mouthwash are prepared, comprising the following ingredients:

| Ingredients (wt%) | Test | Control |
|---|---|---|
| Demineralized water | 78.975 | 79.175 |
| Poloxamer 338 | 0.5 | 0.5 |
| Sodium Saccharin | 0.02 | 0.02 |
| Wheat Hydrolysate (Gluadin W20, BASF) | 0.2 | -- |
| Cetylpyridinium Chloride | 0.04 | 0.04 |
| Sodium Fluoride | 0.05 | 0.05 |
| Potassium Sorbate | 0.05 | 0.05 |
| 99.0% - 101.0% Glycerin | 7.5 | 7.5 |
| Sorbitol (70% solution) | 5.5 | 5.5 |
| Propylene Glycol | 7 | 7 |

(continued)

| Ingredients (wt%) | Test | Control |
| --- | --- | --- |
| Optacool (coolant flavor, Symrise) | 0.025 | 0.025 |
| L-Menthol | 0.02 | 0.02 |
| Peppermint Flavor | 0.12 | 0.12 |

Bovine teeth are cut, ground and polished to get enamel blocks (around 3x3x2 mm). The enamel thickness is 1-2 mm and the dentin thickness is ~1mm thick. The enamel blocks are marked as left (used for treatment) and right side (used as internal control) using pencil. All MH measurements are completed on both left and right side on the enamel surface.

Microhardness (Knoop hardness-KH, 50g load) of sound enamels are measured, and the blocks with KH > 300 are chosen. The blocks then are etched with 30% phosphoric acid for 15sec. The KH are measured as etched.

Before every treatment, the right side of each block is covered with UPVC tape. The right side of the block is used as internal control, so only the left side of the block is exposed to treatment. The individual block is treated with 1 ml designated treatment solution (test or control mouthwash) for 1 minute. After the 1 minutes of treatment is done, the block is rinsed twice with deionized water for 5 minutes at 500 RPM before it is unwrapped and the UPVC tape removed. The block is air-dried on a lint free tissue (Kimwipe), and then incubated at 37°C in 15 ml artificial saline ($MgCl_2$ 0.2 mM, $CaCl_2 \cdot H_2O$ 1mM, HEPES buffer 20 mM, $KH_2PO_4$ 4 mM, KCl 16 mM, $NH_4Cl$ 4.5 mM, NaF 300 ppm, 0.05 wt% $NaN_3$, pH = 7.0, (adjusted with 1 M NaOH)). Thus the left side is exposed to treatment, while the right side is incubated in artificial saline only. The right side of the block is re-wrapped before the next treatment is started.

Four different treatments are evaluated:

A - Test mouthwash: treat and then incubate in artificial saliva overnight, repeat for 7 days.

B - Control mouthwash: treat and then incubate in artificial saliva overnight, repeat for 7 days.

C - Test mouthwash: treat (once), and then incubate in artificial saliva for 7 days.

D - control mouthwash: treat (once), and then incubate in artificial saliva for 7 days

The blocks used in treatment A and B are taken out of artificial saline after the overnight incubation, washed with DI water, air dried, and the treatment repeated for six more days. For the blocks in group C and D, there is no more treatment after the initial treatment, but the fresh saliva is replenished every day. At the end of the protocol, all blocks are taken out, washed with deionized water (twice at 500 rpm for 5 minutes), air-dried, and then stored for the final measurement after the treatment/incubation period was completed.

The microhardness is measured and the remineralization percentage is calculated using equation 1 above. The percent remineralization provided by the treatment with the test versus control mouthwashes is calculated by subtracting the percent remineralization observed in the internal control on the right side from the percent remineralization observed on the left side (i.e., remineralization % of treatment = remineralization % of left side - remineralization % of right).

The blocks treated once per day repeated for 7 days have around 15 % remineralization for the wheat mouthwash and 8.5% remineralization for the placebo mouthwash. The blocks treated only once have a 5.7% remineralization for the wheat mouthwash and 4.4% for the placebo mouthwash. This shows that wheat hydrolysate keeps its enamel repair benefit in mouthwash formula; and the wheat hydrolysate showed better efficacy in repeated treatment

**Claims**

1. An oral care composition comprising:

    a. partially hydrolyzed plant protein, comprising oligo- and polypeptide molecules having a molecular weight distribution of about 500 to about 10000 D;
    b. an orally acceptable carrier,

    for use in a method of repairing or inhibiting dental erosion, promoting dental remineralization, and/or enhancing the anti-cavity effects of fluoride comprising applying to the teeth said oral care composition, wherein the partially hydrolyzed plant protein is present in the composition in an amount of from 0.1 weight % to 3 weight % by total weight of the composition and wherein the partially hydrolyzed plant protein is selected from partially hydrolyzed wheat protein, partially hydrolyzed rice protein and combinations thereof.

2.  The composition for use according to claim 1 wherein the oral care composition further comprises an effective amount of fluoride.

3.  The composition for use according to claim 2 wherein the amount of fluoride is about 100 - 1500 ppm fluoride.

4.  The composition for use according to any foregoing claim wherein the composition is a dentifrice, gel, mouthrinse, tablet, toothpowder, or a chewing gum, optionally wherein the composition is a dentifrice.

5.  A dentifrice comprising

    a. a partially hydrolyzed plant protein comprising oligo- and polypeptide molecules having a molecular weight distribution of about 500 to about 10000 D,
    b. an effective amount of fluoride and
    c. an orally acceptable carrier,

    wherein the partially hydrolyzed plant protein is present in the composition in an amount of from 0.1 weight % to 0.5 weight % by total weight of the composition and wherein the partially hydrolyzed plant protein is selected from partially hydrolyzed wheat protein, partially hydrolyzed rice protein and combinations thereof.

6.  The dentifrice of claim 5 wherein the amount of fluoride is 100 ppm to 1500 ppm.

7.  An oral care composition comprising:

    a. partially hydrolyzed plant protein;
    b. optionally an effective amount of fluoride;
    c. an orally acceptable carrier,

    wherein the partially hydrolyzed plant protein is present in the composition in an amount of from 0.1 weight % to 0.5 weight % by total weight of the composition and wherein the partially hydrolyzed plant protein is selected from partially hydrolyzed wheat protein, partially hydrolyzed rice protein and combinations thereof.


**Patentansprüche**

1.  Mundpflegezusammensetzung, umfassend:

    a. teilweise hydrolisiertes Pflanzenprotein, umfassend Oligo- und Polypeptidmoleküle mit einer Molekulargewichtsverteilung von etwa 500 bis etwa 10000 D;
    b. einen oral akzeptablen Träger,

    zur Verwendung in einem Verfahren zum Reparieren oder Hemmen dentaler Erosion, zum Fördern dentaler Remineralisierung und/oder zum Verstärken der Anti-Hohlraum-Wirkungen von Fluorid, umfassend Auftragen dieser Mundpflegezusammensetzung auf die Zähne, wobei das teilweise hydrolysierte Pflanzenprotein in der Zusammensetzung in einer Menge von 0,1 Gew.-% bis 3 Gew.-% bezogen auf das Gesamtgewicht der Zusammensetzung vorhanden ist und wobei das teilweise hydrolysierte Pflanzenprotein ausgewählt ist aus teilweise hydrolysiertem Weizenprotein, teilweise hydrolysiertem Reisprotein und Kombinationen davon.

2.  Zusammensetzung zur Verwendung gemäß Anspruch 1, wobei die Mundpflegezusammensetzung ferner eine wirksame Menge an Fluorid umfasst.

3.  Zusammensetzung zur Verwendung gemäß Anspruch 2, wobei die Menge an Fluorid etwa 100 - 1500 ppm Fluorid ist.

4.  Zusammensetzung zur Verwendung gemäß einem beliebigen der vorstehenden Ansprüche, wobei die Zusammensetzung eine Zahnpasta, Gel, Mundspülung, Tablette, Zahnpulver oder ein Kaugummi ist, wahlweise wobei die Zusammensetzung eine Zahnpasta ist.

5.  Zahnputzmittel, umfassend

a. ein teilweise hydrolysiertes Pflanzenprotein, das Oligo- und Polypeptidmoleküle mit einer Molekulargewichtsverteilung von etwa 500 bis etwa 10000 D umfasst,

b. eine wirksame Menge an Fluorid und

c. einen oral akzeptablen Träger,

wobei das teilweise hydrolysierte Pflanzenprotein in der Zusammensetzung in einer Menge von 0,1 Gew.-% bis 0,5 Gew.-% bezogen auf das Gesamtgewicht der Zusammensetzung vorhanden ist und wobei das teilweise hydrolysierte Pflanzenprotein ausgewählt ist aus teilweise hydrolysiertem Weizenprotein, teilweise hydrolysiertem Reisprotein und Kombinationen davon.

6. Zahnpasta nach Anspruch 5, wobei die Menge an Fluorid 100 ppm bis 1500 ppm ist.

7. Mundpflegezusammensetzung, umfassend:

a. teilweise hydrolisiertes Pflanzenprotein;

b. wahlweise eine wirksame Menge an Fluorid;

c. einen oral akzeptablen Träger,

wobei das teilweise hydrolysierte Pflanzenprotein in der Zusammensetzung in einer Menge von 0,1 Gew.-% bis 0,5 Gew.-%, bezogen auf das Gesamtgewicht der Zusammensetzung, vorhanden ist und wobei das teilweise hydrolysierte Pflanzenprotein ausgewählt ist aus teilweise hydrolysiertem Weizenprotein, teilweise hydrolysiertem Reisprotein und Kombinationen davon.

**Revendications**

1. Composition de soins buccaux comprenant :

a. protéine végétale partiellement hydrolysée, comprenant des molécules oligo- et polypeptidiques ayant une distribution de poids moléculaire d'environ 500 à environ 10000 D;

b. un support oralement acceptable,

pour une utilisation dans une méthode de réparation ou d'inhibition de l'érosion dentaire, de promotion de la reminéralisation dentaire, et/ou d'amélioration des effets anti-cavité du fluorure comprenant l'application sur les dents de ladite composition de soin oral, dans laquelle la protéine végétale partiellement hydrolysée est présente dans la composition en une quantité de 0,1 % en poids à 3 % en poids par rapport au poids total de la composition et dans laquelle la protéine végétale partiellement hydrolysée est choisie parmi les protéines de blé partiellement hydrolysées, les protéines de riz partiellement hydrolysées et leurs combinaisons.

2. Composition à utiliser selon la revendication 1, dans laquelle la composition de soins bucco-dentaires comprend en outre une quantité efficace de fluorure.

3. Composition à utiliser selon la revendication 2, dans laquelle la quantité de fluorure est d'environ 100 - 1500 ppm de fluorure.

4. Composition à utiliser selon l'une quelconque des revendications précédentes, dans laquelle la composition est un dentifrice, gel, bain de bouche, comprimé, poudre dentifrice ou un chewing-gum, éventuellement dans laquelle la composition est un dentifrice.

5. Dentifrice comprenant

a. une protéine végétale partiellement hydrolysée comprenant des molécules oligo- et polypeptidiques ayant une distribution de poids moléculaire d'environ 500 à environ 10000 D,

b. une quantité efficace de fluorure et

c. un support acceptable par voie orale,

dans laquelle la protéine végétale partiellement hydrolysée est présente dans la composition en une quantité de 0,1 % en poids à 0,5 % en poids par rapport au poids total de la composition et dans laquelle la protéine végétale partiellement hydrolysée est choisie parmi les protéines de blé partiellement hydrolysées, les protéines de riz partiellement hydrolysées et leurs combinaisons.

6. Dentifrice selon la revendication 5, dans lequel la quantité de fluorure est de 100 ppm à 1500 ppm.

7. Composition de soins buccaux comprenant :

    a. protéine végétale partiellement hydrolysée;
    b. éventuellement une quantité efficace de fluorure ;
    c. un support oralement acceptable,

dans laquelle la protéine végétale partiellement hydrolysée est présente dans la composition en une quantité de 0,1 % en poids à 0,5 % en poids par rapport au poids total de la composition et dans laquelle la protéine végétale partiellement hydrolysée est choisie parmi les protéines de blé partiellement hydrolysées, les protéines de riz partiellement hydrolysées et leurs combinaisons.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62248992
- US 62246161
- US 62246163
- US 62246165
- US 20100196287 A1
- US 20060222602 A1